**Europäisches Patentamt**

**European Patent Office**

**Office européen des brevets**

(19)

(11) Veröffentlichungsnummer: **0 289 922 B1**

# EUROPÄISCHE PATENTSCHRIFT

(12)

(45) Veröffentlichungstag der Patentschrift:
19.06.91 Patentblatt 91/25

(51) Int. Cl.⁵: **A61F 2/36**

(21) Anmeldenummer: 88106700.3

(22) Anmeldetag: 27.04.88

(54) **Gelenkprothese und Verfahren zu ihrer Herstellung.**

(30) Priorität: 06.05.87 DE 3715000

(43) Veröffentlichungstag der Anmeldung:
09.11.88 Patentblatt 88/45

(45) Bekanntmachung des Hinweises auf die
Patenterteilung:
19.06.91 Patentblatt 91/25

(84) Benannte Vertragsstaaten:
CH FR GB IT LI

(56) Entgegenhaltungen:
EP-A- 0 065 481
EP-A- 0 163 013
EP-A- 0 243 585
DE-A- 2 851 598
US-A- 4 608 055

(73) Patentinhaber: **Fried. Krupp Gesellschaft mit
beschränkter Haftung
Altendorfer Strasse 103
W-4300 Essen 1 (DE)**

(72) Erfinder: **Bensmann, Günter, Dr. Ing.
Wortbergrode 11
W-4300 Essen 1 (DE)**

(74) Vertreter: **Vomberg, Friedhelm, Dipl.-Phys.
Schulstrasse 8
W-5650 Solingen 1 (DE)**

**Beschreibung**

Die Erfindung bezieht sich auf eine Gelenkprothese mit einem Schaft, der als Hohlkörper ausgebildet ist und aus durchlöchertem Blech besteht und der einen Kragen und einen Ansatz besitzt, der zum Aufsetzen der Gelenkkugel dient.

Derzeit unterscheidet man bei der Endoprothetik zwischen zwei Befestigungsvarianten des Prothesenschaftes im Knochen. Entweder werden die Prothesen mit Hilfe eines Knochenzementes in der vorbereiteten Knochenhöhle befestigt oder es werden sogenannte Zementlosprothesen verwendet.

Bei der Zementiertechnik werden in der Regel gute Anfangserfolge erzielt, da der Zement sich der vorbereiteten Knochenhöhle sehr gut anpaßt. Da der Knochenzement aber nach einer gewissen Zeit versprödet, lockern sich die Prothesen und müssen entfernt werden.

Diesem Nachteil versucht man durch die Entwicklung von Zementlosprothesen zu begegnen. Bei der Zementlostechnik werden oberflächenstrukturierte Prothesenschäfte in die möglichst genau vorgearbeitete Knochenhöhle eingebracht und dort mittels Preßsitz fixiert. Danach muß, um eine dauerhafte Fixierung der Prothese zu erhalten, der Knochen in die Oberflächenstruktur einwachsen. Da die Biegesteifigkeit eines Prothesenschaftes aber wesentlich größer ist als die Biegesteifigkeit des Knochens in den er implantiert wurde, treten bei Belastung der Prothese Relativbewegungen zwischen Knochen und Schaft auf, was zur bindegewebigen Abscheidung des Prothesenschaftes und damit zu Lockerungen führt.

Es ist bereits eine Prothese bekannt (DE-OS 31 20 147), die einen Schaft aufweist, der als Hohlkörper ausgebildet ist, der aus durchlöchertem Blech besteht und der einen Kragen und einen Ansatz besitzt, der zum Aufsetzen der Gelenkkugel dient. In das Innere der Hohlprothese kann nach der Primärfixation zerkleinerte Knochenspongiosa, die während der Operation in ausreichender Menge anfällt, eingeführt werden. Der Hohlschaft der Prothese ist mit einer ausreichenden Anzahl von unterschiedlich großen Löchern versehen, so daß die in den Schaft eingepreßte Spongiosa Kontakt zur Wandung der Knochenhöhle aufnehmen kann und dort anwachsen kann.

Ein wesentlicher Unterschied zur derzeitigen Zementlos-Prothetik besteht folglich darin, daß der Knochen nicht in die Oberflächenstruktur einwachsen muß. Vielmehr kann bei dieser Konstruktion bereits während der Operation der Knochen dahin gebracht werden, wo er benötigt wird. Durch die ausreichende Dimensionierung der Löcher im Prothesenschaft kann Sorge dafür getragen werden, daß der in den Schaft eingebrachte Knochen anwächst und auch auf Dauer ernährt werden kann.

Die Ausbildung des Prothesenschaftes als gelochter Hohlschaft hat ferner den Vorteil, daß durch Anordnung und Dimensionierung der Löcher die Biegesteifigkeit des Schaftes der Biegesteifigkeit des Knochens weitgehend angepaßt werden kann, so daß bei Belastung des Systems Knochen/Prothese Relativbewegungen gerinfügig sind.

Die bekannten gelochten Hohlprothesen (DE-OS 31 20 147) sind jedoch mit vertretbarem technischen Aufwand nicht herstellbar.

Aufgabe der Erfindung ist es, eine Gelenkprothese zu schaffen, die die Vorteile der bekannten Prothese aufweist, aber einfach und mit vertretbarem technischen Aufwand hergestellt werden kann. Diese Aufgabe wird nach der Erfindung mit einer Prothese mit den Merkmalen des kennzeichnenden Teils des Anspruchs 1 gelöst. Von besonderem Vorteil ist es, in dem am oberen Ende des Schaftes angeschweißten Kragen eine zum Inneren des Hohlschaftes führende Öffnung vorzusehen. Diese Öffnung dient dazu, den Hohlschaft mit Knochenspongiosa zu füllen, ohne den Prothesenkopf abzunehmen.

Durch den Aufbau der Prothese nach der Erfindung aus einzelnen, erforderlichenfalls vorgeformten Blechstreifen, die zu einem Hohlschaft durch Schweißen verbunden werden, kann auch der Vorteil erreicht werden, daß die Biegefestigkeit des Schaftes an jeder Stelle der Biegefestigkeit des Knochens für den er vorgesehen ist, an der entsprechenden Stelle angenähert entspricht. Durch die geeignete Anordnung und Dimensionierung der Löcher sowie durch Fräsen oder Schleifen an geeigneten Stellen kann eine verringerte Wandstärke der Blechstreifen erreicht werden.

Die Zeichnungen zeigen ein Ausführungsbeispiel der erfindungsgemäßen Hohlprothese und zwar zeigt

Fig. 1 einen Längsschnitt durch die Prothese,

Fig. 2 einen Querschnitt nach der Linie II-II in Fig. 1 und

Fig 3 einen vergrößerten Längsschnitt durch die Prothese nach Fig. 1.

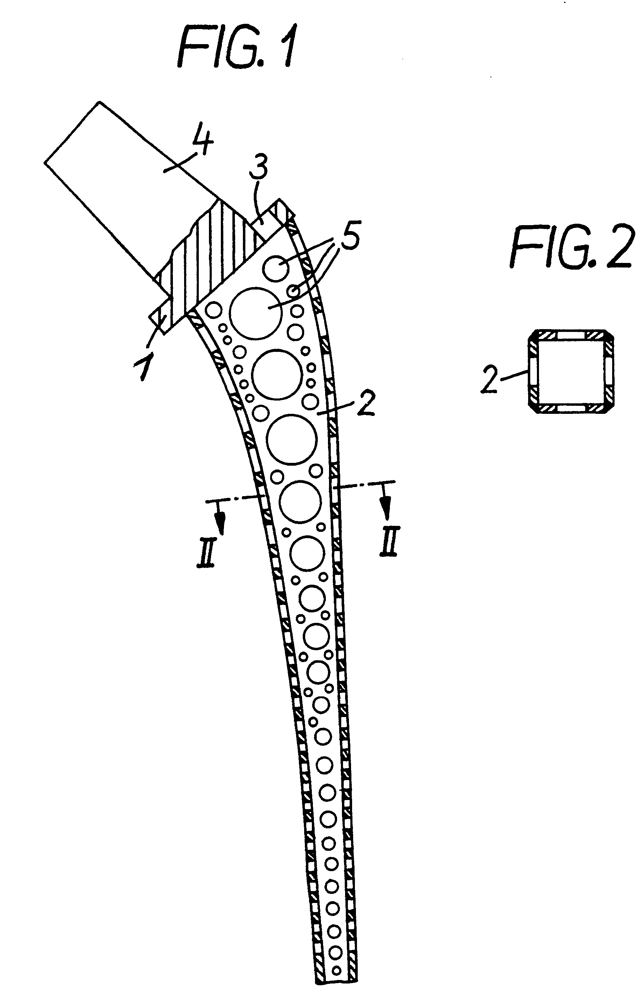

Der in Fig. 1 dargestellte Querschnitt der Prothese zeigt den Kragen 1, der an den Hohlschaft 2 angeschweißt ist, mit der Öffnung 3 und dem Ansatz 4 zur Aufnahme der Gelenkkugel. Der Hohlschaft 2 besteht in diesem Ausführungsbeispiel aus vier geeignet zugeschnittenen und ggf. vorgeformten Blechstreifen aus CoCrMo, die miteinander verschweißt sind. Der Querschnitt des Hohlschaftes ist in Fig. 2 dargestellt. Durch die Öffnung 3 im Kragen 1 kann der Hohlschaft 2 mit Knochenspongioasa verfüllt werden. Die Bleche sind in der Weise mit Löchern 5 verschiedener Anordnung und Größe versehen, daß die Biegesteifigkeit des Hohlschaftes 2 möglichst genau mit der Biegesteifigkeit des Knochens übereinstimmt. Die Berechnung der Anordnung und Verteilung der Löcher 5 für eine bestimmte Biegesteifigkeit kann z.B. durch die Methode der Finiten-Elemente ermittelt werden.

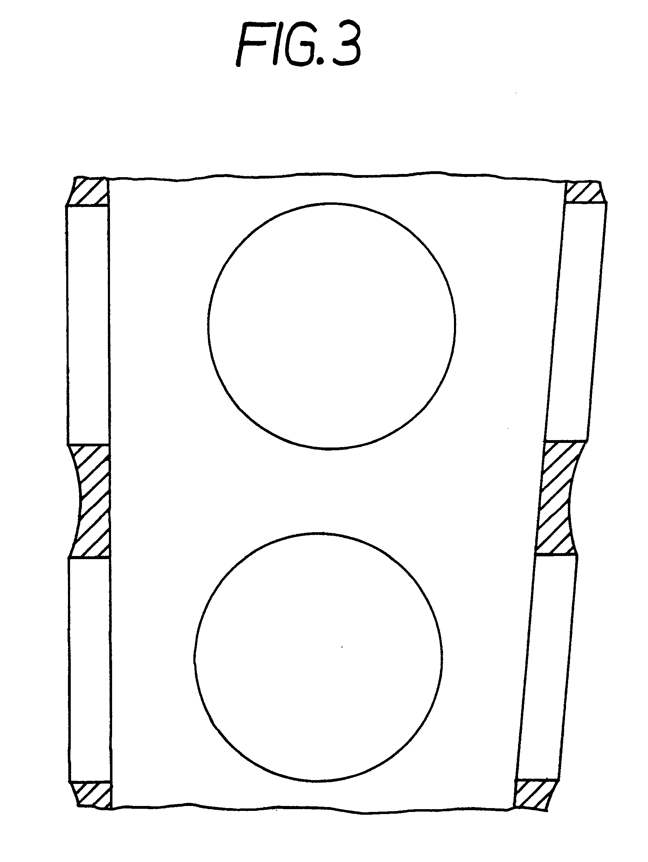

Entsprechend der Weiterentwicklung der erfindungsgemäßen Prothese zeigt Fig. 3 in einem Ausschnitt aus dem Längsschnitt beispielhaft eine mögliche Anordnung von Stellen durch Fräsen oder Schleifen verringerter Blechstärke zur Feinabstimmung der Biegesteifigkeit der Prothese.

## Ansprüche

1. Gelenkprothese mit einem als Hohlkörper ausgebildeten Schaft (2), der aus gelochtem Blech besteht und einen Kragen (1) mit einem Ansatz (4) besitzt, der zum Aufsetzen der Gelenkkugel dient, **dadurch gekennzeichnet,** daß der Schaft aus einzelnen, erforderlichenfalls vorgeformten, gelochten Blechstreifen besteht, die zur Bildung des Hohlschaftes miteinander und mit dem am oberen Ende des Schaftes angeordneten mit einer zum Inneren des Hohlschaftes führenden Öffnung (3) versehenen Kragen durch Schweißen verbunden sind, wobei durch die Öffnung nach der Implantation gemahlene bzw. zerkleinerte Knochenspongiosa eingebracht werden kann.

2. Gelenkprothese nach Anspruch 1, dadurch gekennzeichnet, daß die Blechstreifen, aus denen der Hohlschaft (2) gebildet ist, über die Fläche unterschiedlich stark sind.

3. Gelenkprothese nach den Ansprüchen 1 und 2, dadurch gekennzeichnet, daß die Wandstärke der Blechstreifen an verschiedenen Stellen und erforderlichenfalls in unterschiedlichem Ausmaß durch Fräsen oder Schleifen verringert ist.

4. Gelenkprothese nach den Ansprüchen 1 bis 3, dadurch gekennzeichnet, daß die Biegefestigkeit des Schaftes an jeder Stelle der Biegefestigkeit des Knochens, für den er vorgesehen ist, an den entsprechenden Stellen angenähert entspricht.

5. Verfahren zur Herstellung einer Hohlprothese nach den Ansprüchen 1 bis 4, dadurch gekennzeichnet, daß für die vorgegebene Biegesteifigkeit die Blechstreifen vor oder nach dem Zuschnitt mit Löchern bestimmter Größe in bestimmter Anordnung versehen werden und/oder die Blechstärke über die Fläche an verschiedenen Stellen in verschiedenem Ausmaß durch Fräsen oder Schleifen verringert wird, daß die Blechstreifen dann ggf. vorgeformt werden, und daß sie zur Bildung eines Hohlschaftes (2) miteinander und mit dem Kragen (1) mit Ansatz (4) zum Aufsetzen der Gelenkkugel durch Schweißen verbunden werden.

6. Gelenkprothese nach den Ansprüche 1 bis 4 oder Verfahren nach Anspruch 5, dadurch gekennzeichnet, daß als Werkstoff CoCrMo-, CoNiCrMo-, Titanlegierungen, Tantal oder Niob verwendet wird.

7. Gelenkprothese nach den Ansprüchen 1 bis 4 oder Verfahren nach den Ansprüchen 5 und 6, dadurch gekennzeichnet, daß die Durchmesser der Löcher in den den Hohlschaft bildenden Blechen ausreichend dimensioniert sind, damit die in den Hohlraum eingebrachte Spongiosa in direkten Kontakt mit der Spongiosa des Knochens treten kann.

## Claims

1. A joint prosthesis having a shaft (2) taking the form of a hollow body of perforate sheet metal having a collar (1) with an attachment (4) on which the ball of the joint can be placed, characterized in that the shaft consists of individual, if necessary preshaped perforate sheet metal strips which are connected by welding to one another to form the hollow shaft and to the collar, which is formed with an opening (3) extending to the inside of the hollow shaft and serves for the introduction of ground or comminuted bone spongiosa after the implantation.

2. A joint prosthesis according to claim 1, characterized in that the sheet metal strips from which the hollow shaft (2) is formed differ in thickness over the surface.

3. A joint prosthesis according to claims 1 and 2, charaterized in that the wall thickness of the sheet metal strips is reduced by milling or grinding at different places and if necessary to a varying extent.

4. A joint prosthesis according to claims 1 to 3, characterized in that at every place the bending strength of the shaft substantially corresponds at the corresponding places to the bending strength of the bone for which the shaft is intended.

5. A process for the production of a hollow prosthesis according to claims 1 to 4, characterized in that prior to or following cutting to size, the sheet metal strips are formed for the given bending strength with holes of predetermined size in a predetermined arrangement and/or the thickness of the sheet metal is reduced over the surface by milling or grinding at different places to a varying extent, whereafter the sheet metal strips are if necessary preshaped and connected by welding to another to form a hollow shaft (2) and to the collar (1) with the attachment (4) on which the ball of the joint can be placed.

6. A joint prosthesis according to claims 1 to 4 or the process according to claim 5, characterized in that the material used is CoCrMo, CoNiCrMo or titanium alloys, tantalum or niobium.

7. A joint prosthesis according to claims 1 to 4 or the process according to claims 5 and 6, characterized in that the diameters of the holes in the metal sheets forming the hollow shaft are adequately dimensioned to enable the spongiosa introduced into the cavity to enter into direct contact with the spongiosa of the bone.

## Revendications

1. Prothèse d'articulation avec une broche (2) réalisée sous la forme d'un corps creux, et qui est constituée de tôle perforée et qui possède une collerette (1) et une saillie (4) qui sert à fournir l'appui de la boule d'articulation, caractérisée en ce que la broche est constituée de bandes de tôle séparées, perforées et mises en forme selon les besoins, qui sont raccordées par soudage entre elles pour former la broche creuse et avec la collerette, disposée à l'extrémité supérieure de la broche et munie d'une ouverture (3) conduisant à l'intérieur de la broche creuse, étant entendu que de la matière spongieuse de l'os, moulue ou fragmentée, peut être introduite par l'ouverture après l'implantation.

2. Prothèse d'articulation suivant la revendication 1, caractérisée en ce que les bandes de tôle, dont est formée la broche creuse (2), ont, sur leur surface, différentes épaisseurs.

3. Prothèse d'articulation suivant la revendication 1 et la revendication 2, caractérisée en ce que l'épaisseur de paroi des bandes de tôle est réduite, par fraisage ou meulage, en plusieurs endroits et dans des mesures différentes suivant les besoins.

4. Prothèse d'articulation suivant les revendications 1 à 3, caractérisée en ce que la rigidité de la broche à la flexion correspond en chaque point sensiblement à la rigidité à la flexion de l'os, pour lequel elle est prévue.

5. Procédé de fabrication d'une prothèse creuse suivant les revendications 1 à 4, caractérisé en ce que, pour la rigidité donnée, les bandes de tôle, avant ou après la découpe, sont munies de trous de grandeur déterminée suivant une disposition déterminée et/ou l'épaisseur de la tôle est réduite, en différents endroits de sa surface, d'une quantité différente, par fraisage ou meulage, en ce que les bandes de tôle sont ensuite, le cas échéant, mises en forme et en ce qu'elles sont raccordées entre elles par soudage pour former une broche creuse (2) et avec la collerette (1) comportant une saillie (4) pour la mise en place de la sphère de l'articulation.

6. Prothèse d'articulation suivant les revendications 1 à 4 ou procédé suivant la revendication 5, caractérisée en ce que, comme matière, on utilise des alliages de cobalt-chrome-molybdène, cobalt-nickel-chrome-molybdène, de titane, du tantale, ou du niobium.

7. Prothèse d'articulation suivant les revendications 1 à 4 ou procédé suivant les revendications 5 et 6, caractérisé en ce que les diamètres des trous dans les tôles formant la broche creuse sont dimensionnés de façon suffisante pour que l'os spongieux introduit dans la cavité puisse entrer en contact direct avec la matière spongieuse de l'os.

# FIG. 1

# FIG. 2

# FIG.3